# EUROPEAN PATENT SPECIFICATION

(11) **EP 1 774 944 B1**
(45) Date of publication and mention of the grant of the patent: **26.01.2011**
(21) Application number: 06021323.8
(22) Date of filing: 11.10.2006
(51) Int. Cl.: A61G 5/10

(54) **Wheelchair control display in particular with infrared capability, wheelchair with a control display and method for setting up a wheelchair control**
Rollstuhlsteuerungsanzeige, insbesondere mit Infrarotfähigkeit, Rollstuhl mit einer Steuerungsanzeige und Verfahren zum Aufbau einer Rollstuhlsteuerung
Affichage de commande de fauteuil roulant en particulier à capacité infrarouge, fauteuil roulant comprenant un affichage de commande et procédé de paramétrage d'une commande de fauteuil roulant

(30) Priority: 11.10.2005 US 725393 P; 11.10.2005 US 725570 P
(43) Date of publication of application: 18.04.2007
(73) Proprietor: Sunrise Medical HHG Inc., Longmont, Colorado 80503 (US)
(72) Inventor: Koerlin, James M., Broomfield, CO 80020 (US)
(74) Representative: Weber-Bruls, Dorothée

(56) References cited:
- EP-A- 1 479 362
- WO-A-94/00832
- US-A- 5 245 558
- US-A1- 2005 075 758
- US-A1- 2005 076 308

## Description

### BACKGROUND OF INVENTION

The present invention is generally related to a wheelchair with a control display and a method for setting up a wheelchair control.

It is not easy to set up and interact with an environmental device via conventional wheelchairs. What is needed is a manner in which a wheelchair occupant may more easily interact with environmental devices.

US 2005/0076308 A I discloses a display for an electronic control system for a personal mobility vehicle, wherein the display has a customised menu structure having one or more menu selection items that are unique to a particular user of the vehicle. The control system includes various modules, such as hand control modules. Visual feedback or display modules may provide visual feedback to the user about the control system. Environmental control modules may be used to allow the user to control devices or accessories external to the vehicle, for example computers or room lights, via the input device of the control system. The environmental control module translates user inputs to control commands to operate the external device.

### SUMMARY OF INVENTION

The present invention is directed to the control of environmental devices by wheelchairs.

A wheelchair control display may comprise a modal screen with one or more features corresponding to functional inputs of a user input device. Each of the features may be tagged with a command associated with an environmental device. When one of the features is selected, a signal associated with the.tag may be transmitted to the environmental device.

The present invention provides a wheelchair in line with claim 1.

A wheelchair that controls environmental devices, thus, may comprise a chassis, one or more ground engaging wheels, a user input device and a control display. The wheels are provided for supporting the chassis for movement in relation to a supporting surface (i.e., the floor or the ground). The wheels may include one or more driven wheels and one or more non-driven caster wheels. The one or more driven wheels may be driven by one or more drive motors. The input device may be supported in relation to the chassis. The control display may be supported in relation to the chassis and may comprise a _ modal screen with one or more control commands corresponding to control functions of an environmental device. The control commands may be selected by the input device to operate the environmental device.

A method for setting up a wheelchair control display is also provided by the present invention in line with claim 4.

The method for setting up a wheelchair control system may, in line with claim 5, comprise the steps of entering on a wheelchair control display a code comprised of one or more digits and/or characters by searching through a string or array of the digits and/or characters using at least a first control command, and selecting one of the digits or characters by using at least a second control command.

### DESCRIPTION OF THE DRAWINGS

Fig. 1 is a side elevational view of an exemplary power wheelchair.

Fig. 2 is a top plan view of an exemplary hand control.

Fig. 3 is a front elevational view of an exemplary control display.

Fig. 4 is a diagrammatic representation of an exemplary driver menu.

Fig. 5 is a diagrammatic representation of an exemplary main menu.

Fig. 6 is a diagrammatic representation of an exemplary expansive menu tree.

Fig. 7 is a rear elevational view of the control display shown in Fig. 3.

Fig. 8 is an environmental, side elevational view of the wheelchair shown in Fig. 1 with signals transmitted therefrom.

Figs. 9A-9G are diagrammatic representations depicting exemplary screen menu instructions for setting up the control display to function as a universal remote control unit for an environmental device.

Figs. 10A-10C are diagrammatic representations depicting exemplary screen menu instructions for setting up the control display to function as a universal remote control unit for an environmental device using a multi digit code.

Fig. 11 is a perspective view of a remote control training the control display shown in Fig. 7.

Figs. 12A-12B are diagrammatic representations depicting exemplary screen menu instructions for programming functions of the control display to operate an environmental device.

Figs. 13A-13F are diagrammatic representations depicting exemplary screen-menu instructions for locating control functions of an environmental device.

Fig. 14 is a diagrammatic representation of an exemplary mode menu for controlling an environmental device.

### BRIEF DESCRIPTION OF THE INVENTION

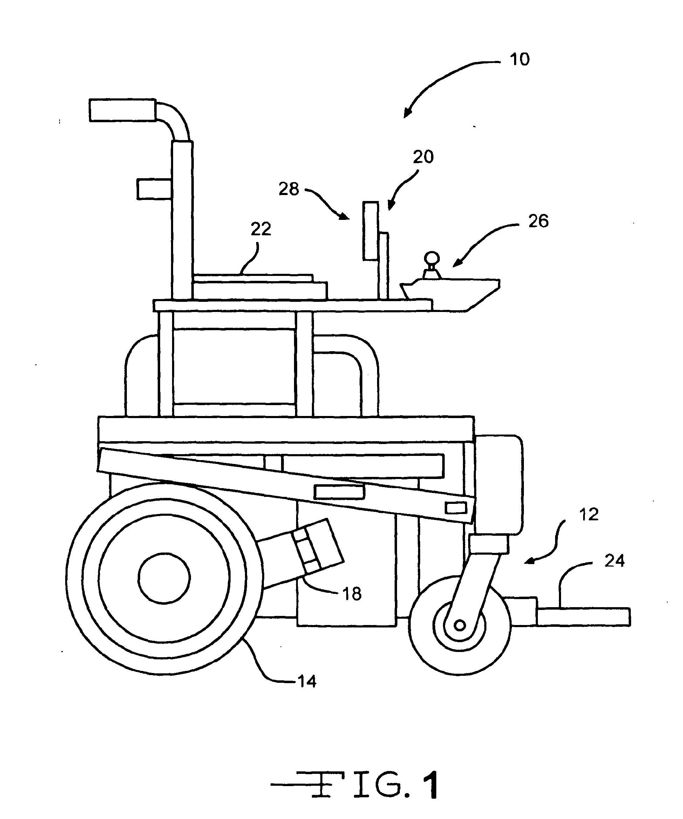

Referring now to the drawings, there is illustrated in Fig. 1 a power wheelchair, generally indicated at 10. The wheelchair 10 may comprise a chassis, which may be inclusive of a frame 12, and which may be supported for movement in relation to a supporting surface (i.e., the floor or the ground) by one or more ground engaging wheels, such as the driven wheels 14 and the non-driven caster wheels 16 shown. The driven wheels 14 may be driven by one or more drive motors 18. The chassis is dimensioned and configured to support various wheelchair components, such as but not limited to a battery tray (not shown) for supporting one or more batteries for providing power to the wheelchair 10, a wiring assembly for supplying power to, and providing communication between, various electronic components of a wheelchair control system and optional electronics, and a seat assembly 20 for supporting a wheelchair occupant. The seat assembly 20 may be of the type that tilts and/or lifts and reclines, and preferably has opposing armrests 22 for supporting the wheelchair occupant's arms and leg rests 24 for supporting the wheelchair occupant's legs. The armrests 22 may support for attachment one or more user input devices, such as the user input device 26 and the control display 28 shown. The various electronic components may include a motor control for controlling the drive motors 18 and various other general functions of the wheelchair 10, a specialty input module for controlling switch-type inputs (e.g., Sip-and-Puff, ASL, Switch-It and Tash discrete switches, and a head control), a multi actuator control (MAC) for controlling one or more actuators (e.g., seat tilt, shear, lift and recline actuators and leg rest actuators), and an environmental control module (ECM) for interfacing with environmental devices, including but not limited to infrared devices, radio frequency devices, or other wireless devices, including but not limited to those using Bluetooth ® technology, of Bellevue, Washington, USA.

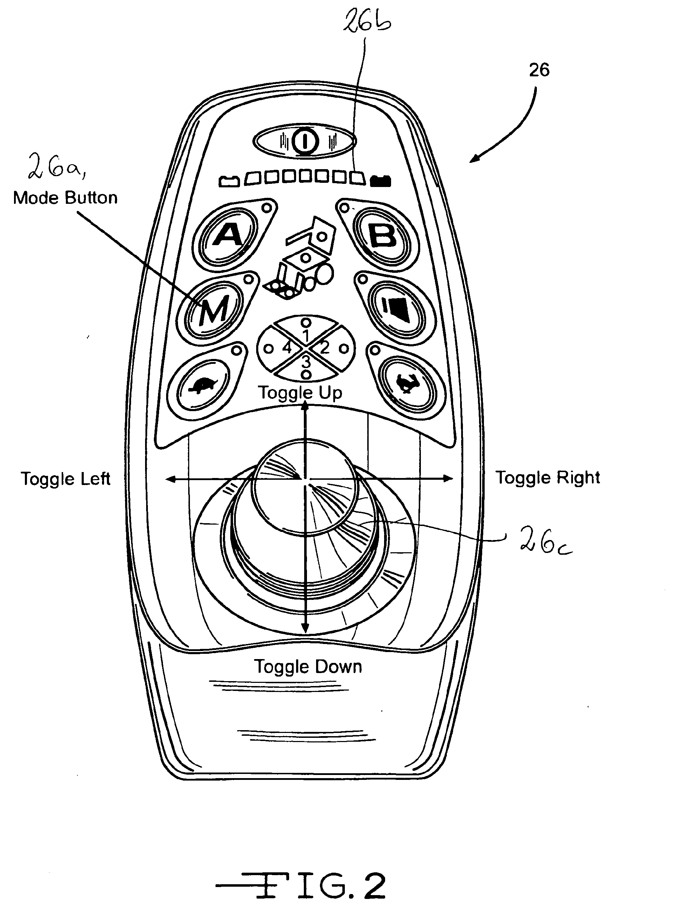

In Fig. 2, there is illustrated an exemplary user input device 26, which is in the form of a multi-button joystick assembly having on/off, mode and horn buttons 26a as well as a joystick 26b, although other user input devices, including switch-type inputs (e.g., Sip-and-Puff, ASL, Switch-It and Tash discrete switches, and a head control), may be employed. The description that follows will refer to the mode button 26a with which the invention may be practised, and joystick toggle directions or commands, such as the up (i.e., forward), down (i.e., reverse), left and right toggle directions shown. Similar toggle directions or commands may be achieved with other inputs (e.g., switches or buttons). Each selected operating mode or function is discernible via indicators 26c.

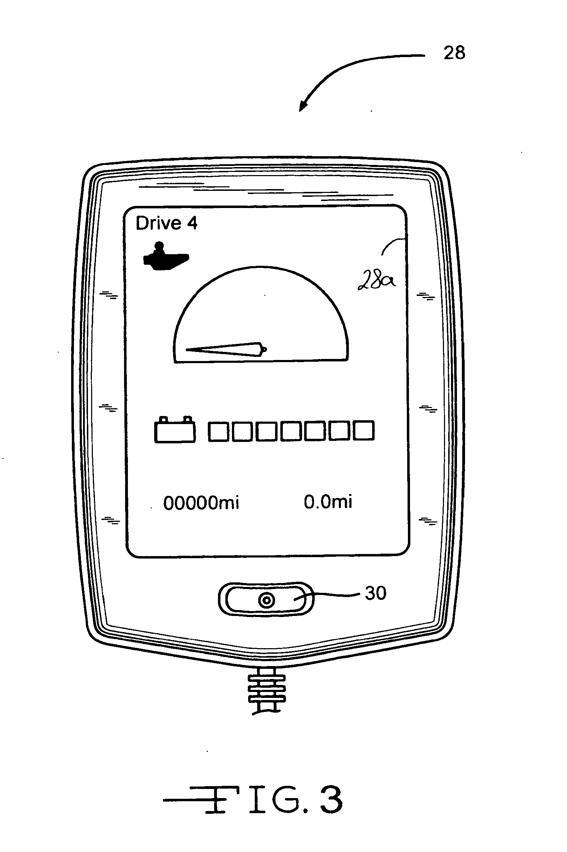

In Fig. 3, the front of the control display 28 is illustrated as having a power switch 30, which turns on and off the control display 28 and preferably the power wheel- .. chair 10. When the control display 28 is turned on, there may appear a drive display that comprises a ready-to-drive screen. This screen 28a may be like a dashboard that may indicate the current drive profile, the type of user input device being used - in this case, a joystick, a speedometer, an odometer and a trip odometer, a battery indicator, and a speed tick mark (not shown), which indicates the maximum speed available in the current drive profile. When this screen 28a is displayed, movement of the hand control 26 causes the wheelchair 10 to move. The control display 28 may provide real-time feedback about the wheelchair's performance to allow the wheelchair occupant to more easily gauge the operation of the wheelchair 10. To move to a driver menu, simply press the mode button 26a.

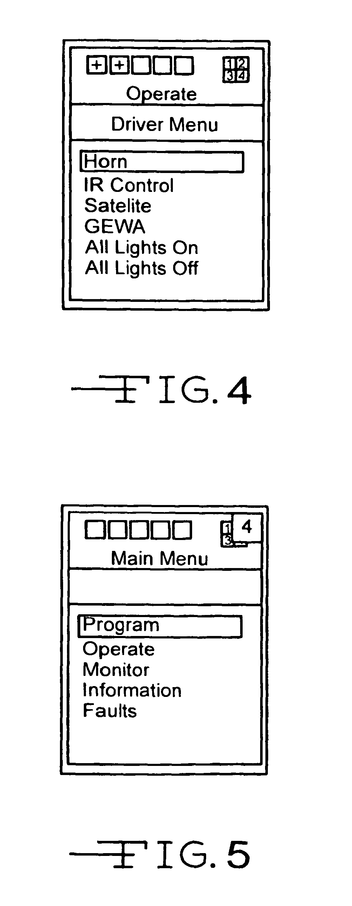

The driver menu, as is illustrated in Fig. 4 and provided on the screen 28a, comprises a list of "shortcuts" to most commonly used non-driving related menu items or functions by the wheelchair occupant. From this menu, the wheelchair occupant can return to the drive display by simply pressing the mode button 26a or quickly toggling the joystick 26c to the left, or if the wheelchair 10 is provided with a timed function, by waiting for a predetermined amount of time to lapse. To enter a main menu, toggle the joystick 26c to the left and hold the joystick 26c in this position for a predetermined period of time (e.g., five seconds).

The main menu, as illustrated in Fig. 5 and provided on the screen 28a, may be the starting point of the electronic menu tree and may contain all the available main menu selection items accessible through the control display 28. The available menu selection items in the illustrated main menu include program, operate, monitor, information, and faults. To return to the drive menu, press the mode button 26a, toggle the joystick 26c to the left (or if a timed function is provided, wait for a predetermined amount of time to lapse).

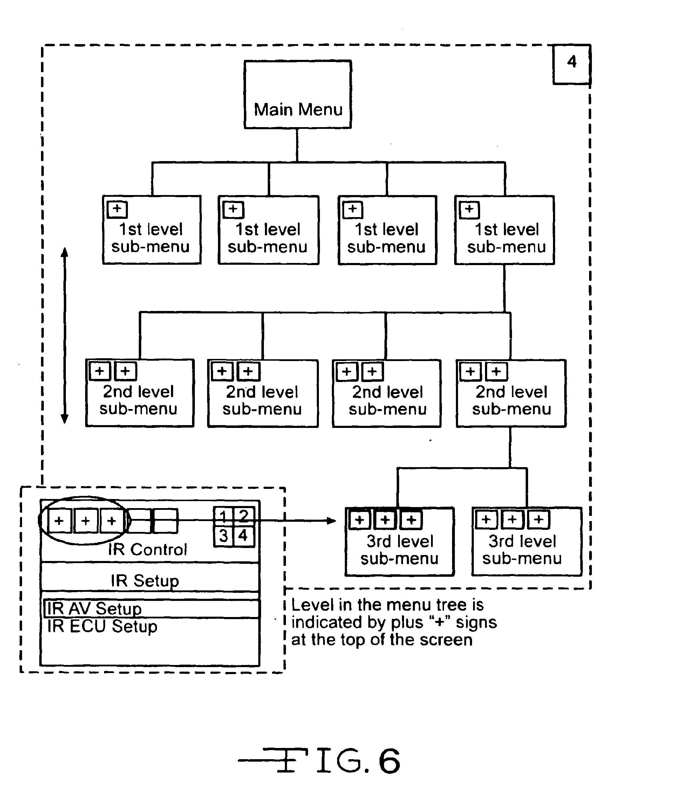

The main menu may be the top menu in an expansive menu tree, as illustrated in Fig. 6. To navigate or move up or down the menu screen, simply toggle the joystick 26c up or down. Similar up or down commands can be achieved with other hand controls or inputs. To move up and down a menu tree (illustrated in Fig. 5), simply toggle the joystick 26c left or right. The level in the menu tree may be indicated by a plus sign at the upper top or another location of the display screen 28a, as illustrated in ' ' the drawings.

There may be different user access levels in the control display 28. One level (i.e., driver and faults) may allow the wheelchair occupant to access the driver menu and fault codes. Another level (i.e., operate and faults) may allow the wheelchair occupant to operate the wheelchair seat as well as switch-type inputs (e.g., Sip-and-Puff, ASL, Switch-It and Tash discrete switches, and a head control). Another level (i.e., operate, monitor, information and faults) may allow wheelchair functions to be monitored. Yet another level (i.e., program, operate, monitor, information and faults) may allow basic and more advanced wheelchair functions to be programmed.

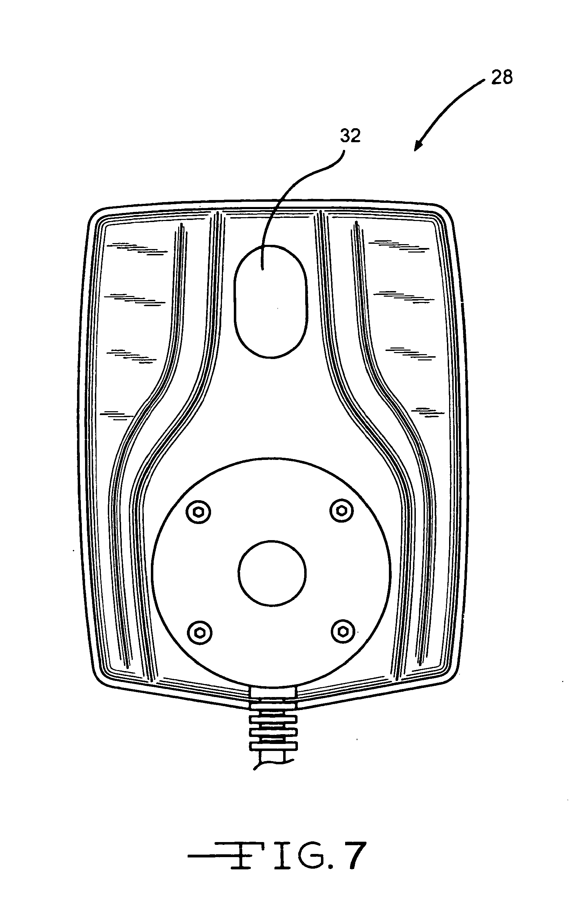

As illustrated in Fig. 7, the back of the control display 28 may have a transceiver 32, which by example is an infrared transceiver. Though the transceiver 32 is provided on the back of the control display 28, it may be provided elsewhere on the wheelchair 10. The transceiver 32 may permit the control display 28 to function as a universal remote control unit for emitting infrared command signals to infrared receptive devices, such as but not limited to audio visual (AV) devices, for example, televisions (TV), set top boxes (STB) (e.g., cable and satellite dish receivers), high fidelity equipment (e.g., tuners and amplifiers, receivers, etc.), digital versatile disk players (DVD), digital video recorders (DVR) (e.g., TIVO, trademark of TIVO, Inc., of Sunnyvale, California, U.S.A.), video cassette recorders (VCR), and compact disk players (CD).

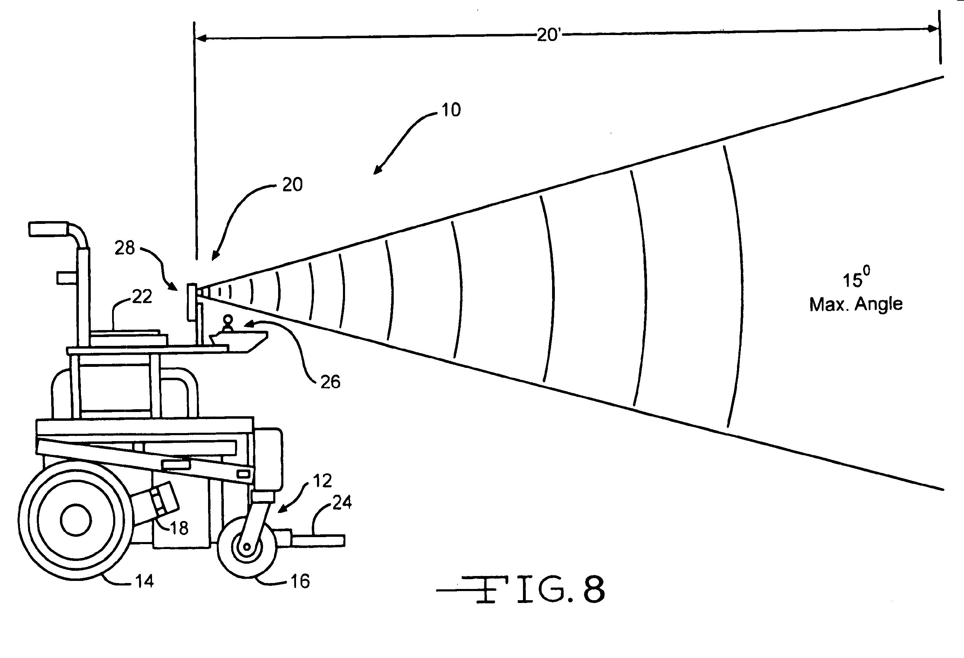

The infrared functions of the control display 28 work by broadcasting or emitting infrared control signals from the transceiver 32 to an infrared receptive device. The infrared control signals emitted from the infrared transceiver 32 preferably spread out at an angle of about 15 degrees, as illustrated in Fig. 8, and have a strong reception at distances up to 20 feet, although this may vary according to the transceiver employed.

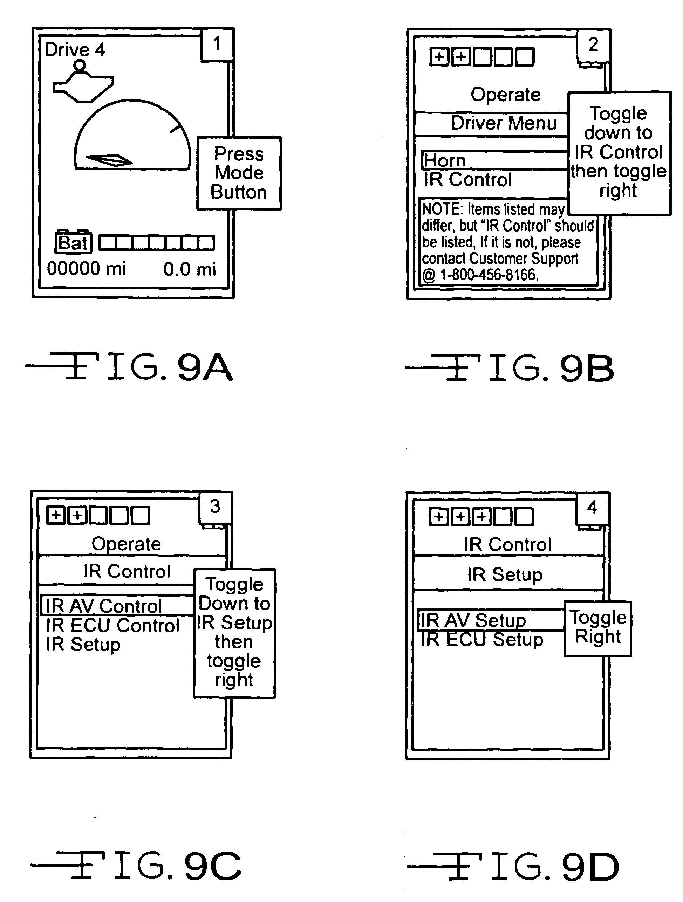

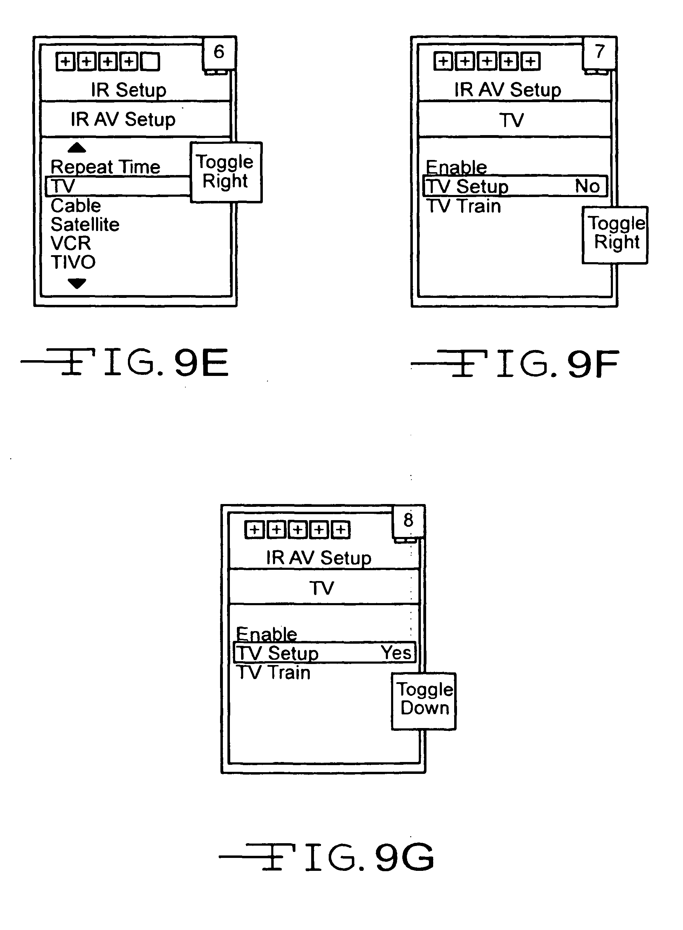

In Figs. 9A-9G, there are illustrated exemplary screen menu instructions for setting up the control display 28 to function as a universal remote control unit for an infrared receptive device. In these screen instructions, the infrared receptive device is a television. In this example, the control display 28 is set up from the drive menu shown in Fig. 9A by first pressing the mode button 26a. This would open the driver menu shown in Fig. 9B, as described above. The wheelchair occupant would then, with the joystick 26c, toggle down to the IR control menu selection item and toggle to the right. This would open the IR control sub-menu shown in Fig. 9C. The wheelchair occupant would then toggle down to the IR AV control menu selection item and again toggle to the right. This would open the IR AV control sub-menu shown in Fig. 9D. From the IR AV control sub-menu, the wheelchair occupant can toggle right to open the IR AV setup sub-menu shown in Fig. 9E. The wheelchair occupant would then toggle down to the menu selection item corresponding to the device the wheelchair occupant wishes to set up, in this example, the TV menu selection item, and then toggle to the right. This would open the TV sub-menu shown in Fig. 9F, In the TV sub-menu, remote operation of an IR receptive device is activated by toggling to the right at the enable menu selection item to change the enable status to "yes," as indicated in Fig. 9G. Once the enable status is changed, the wheelchair occupant may then toggle down to select either the TV setup menu selection item or the TV train menu selection item. To disable the remote operation, the enable status may be changed back to "no." It should be appreciated that the labels and navigation scheme described above are provided by example, and that the invention may be practiced with other labels and navigation schemes.

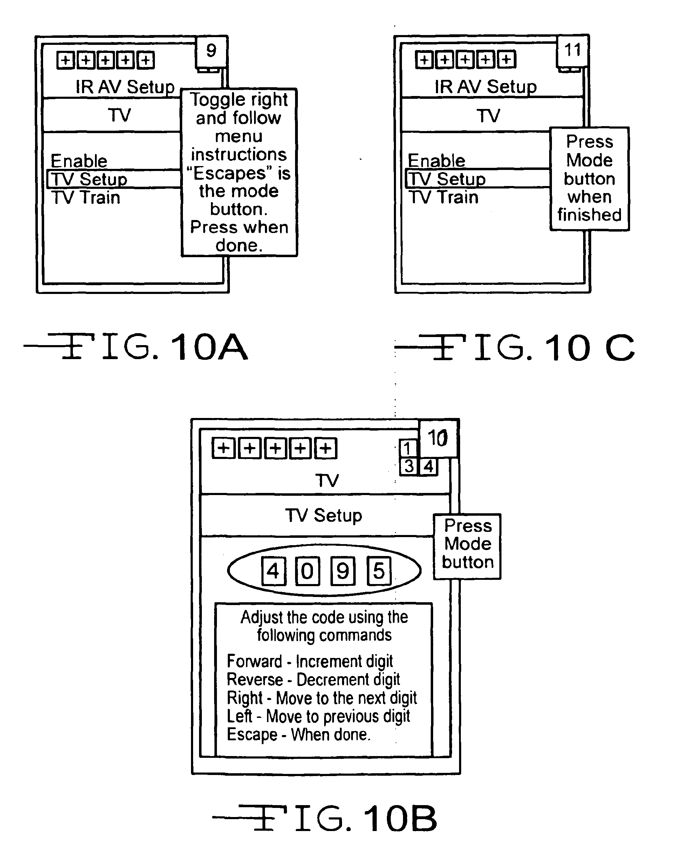

In Figs. 10A-10C, there are illustrated exemplary screen menu instructions for setting up the control display 28 to function as a universal remote control unit for a television using a multi (e.g., four) digit code. Each manufacturer has its own four digit setup codes. Other environmental devices (e.g., set top boxes, high fidelity equipment, digital versatile disk players, digital video recorders, video cassette records, and compact disk players) can be set up with similar four digit codes. In the TV sub-menu shown in Fig. 10A, toggle down to the TV setup menu selection item and then toggle to the right. This would open the TV setup menu shown in Fig. 10B. The wheelchair occupant would then follow the instructions appearing on the display screen for entering the four digit code, as shown in the drawing. For example, toggling up increments a digit, toggling down decrements a digit, toggling left moves to the left to the next digit, and toggling right moves to the right to the previous digit, In the illustrated example, the four digit code is "4095." Once the four digit code has been entered, the wheelchair occupant can escape from the TV setup menu by pressing the mode button 26a, which is described above with reference to Fig. 2, Continue to press the mode button to return back to the drive menu, or if a timed function is provided, wait for a predetermined amount of time to lapse. It should be appreciated that the labels, instructions and navigation scheme described above are provided by example, and that the invention may be practiced by other labels, instructions and navigation schemes.

If the four digit codes do not work after going through the TV setup above, or if the device does not have a four digit code, the control display 28 may be trained. Most devices with infrared capability have a handheld infrared remote control. Training essentially programs the control display 28 to mimic infrared codes that are transmitted by the manufacturer's remote control.

Whenever a button on a remote control 33 is pressed, the remote control 33 transmits an infrared code corresponding to that button's function, as illustrated in Fig, 11. The infrared transceiver 32 on the back of the control display 28, or located elsewhere on the wheelchair 10, may both transmit and receive infrared codes. Aiming a remote control 33 at the back of the control display 28 and pressing the button for a particular function (e.g., Channel Up) transmits or beams that button's infrared code into the transceiver 32. These infrared codes can be logged as functions displayed on the control display TV train sub-menu. Once logged, any time these functions are selected in the control display menu, the infrared codes are transmitted from the control display 28.

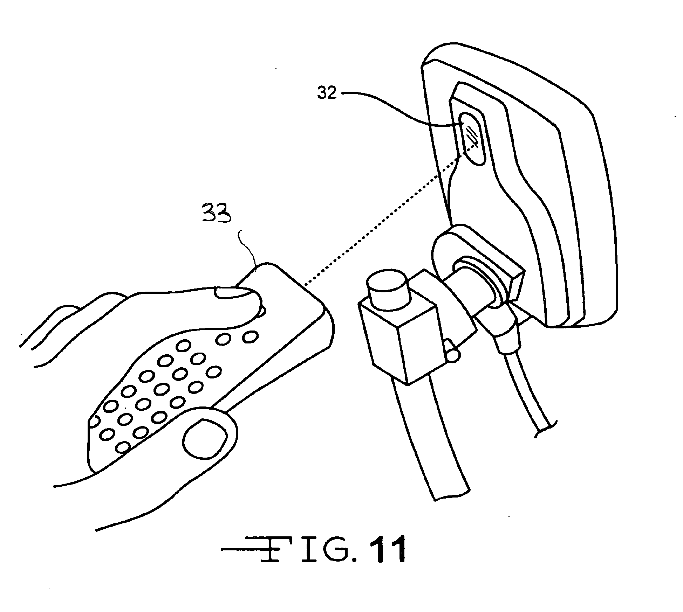

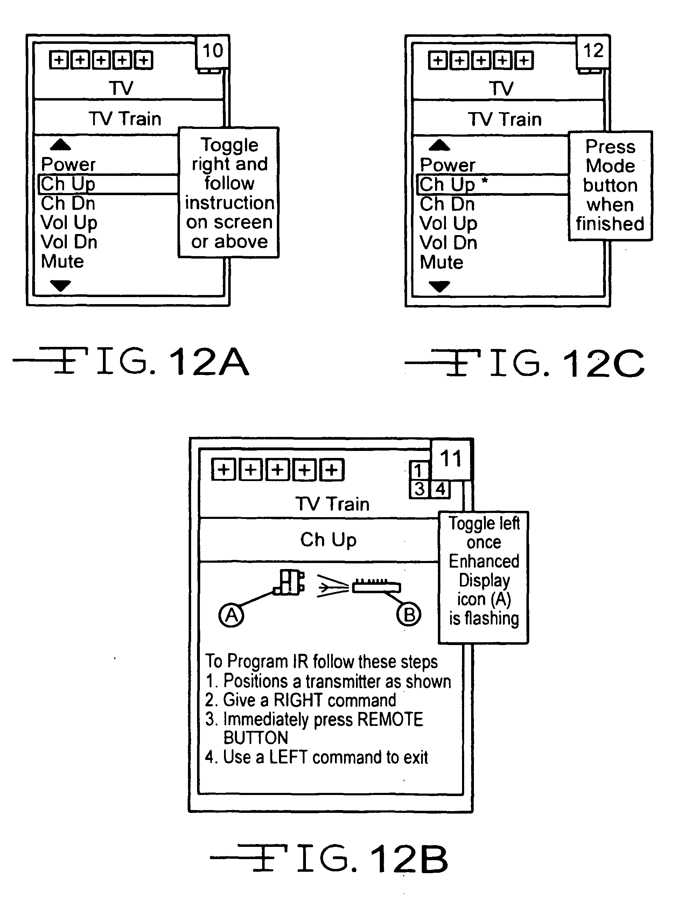

In Fig. 12A, there are illustrated exemplary screen menu instructions for programming the control display 28. Toggle right at the highlighted function to train that function (e.g., Ch Up). This will open a display screen for training that function. The display can provide by example instructions for logging an infrared code, as illustrated in Fig. 12B. The instructions may, for example, prompt the wheelchair occupant to position the remote control 33 in front of the transceiver 32, preferably about 8-12 inches from the back of the control display 28, as illustrated in Fig. 11. Quickly toggle the joystick 26c to the right to prompt the transceiver 32 to receive an infrared signal. Press the channel up function button on the remote control 33 to transmit the infrared code to the transceiver 32. Once the control display 28 is programmed, toggle left to exit. An asterisk or other indicia may appear next to the programmed function to indicate that it has been "trained. Press the mode button 26a to exit the TV train menu when training is complete. It should be appreciated that the labels, instructions, and navigation scheme described above are provided by example, and that the invention may be practiced with other labels, instructions and navigation schemes.

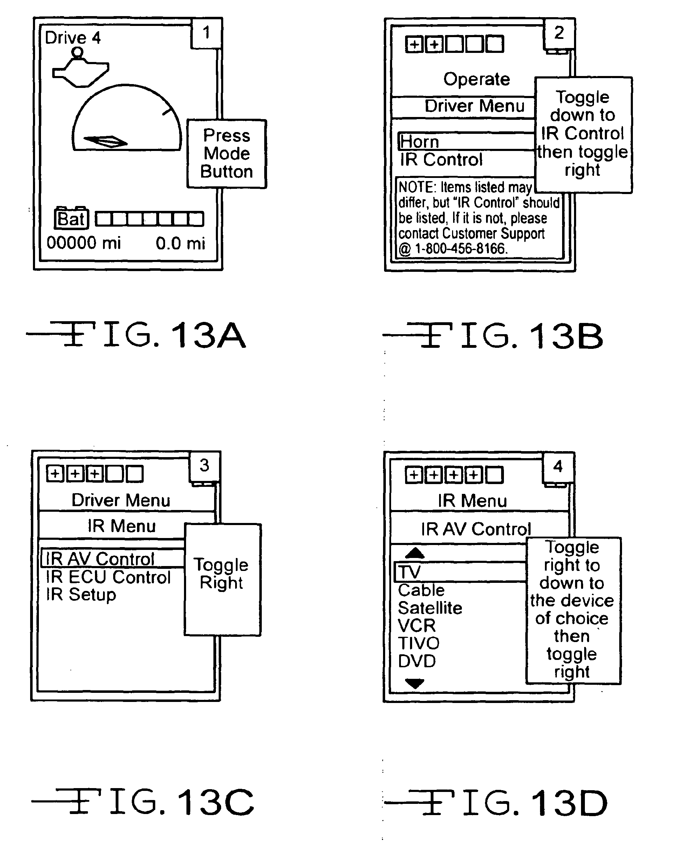

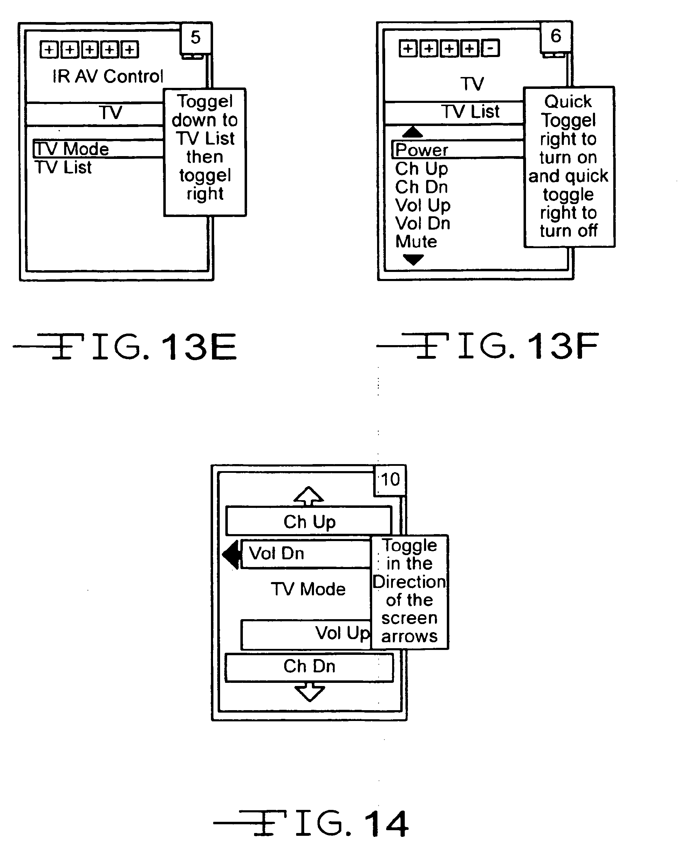

Once an infrared receptive device has been set up for use, operation is simply a matter of toggling to the appropriate screen menu. For example, in Figs. 13A-13F there are illustrated exemplary screen-menu instructions for locating the control functions of an infrared receptive device, in this example, control functions of the television. The drawings provide a roadmap that starts from at the drive menu (illustrated in Fig. 13A), which appears when the wheelchair 10 is turned on. To locate a list of television control functions, simply toggle down and right through the driver menu and IR menu to the IR AV control menu (illustrated in Figs 13A to 13C). In the IR AV control menu, toggle down to TV and then toggle right to open the TV menu selection item (illustrated in Figs 13C and 13D). Next, toggle down to TV list and then again toggle right to open the TV list menu selection item (see Fig. 13E). This will open a list of television control functions (e.g., Power, Ch Up, Ch Dn, Vol Up, Vol Dn, Mute, etc.) as shown in Fig. 13F. Generic labels may be provided in the control list. These labels may be programmed to function as controls for control functions not found in the list. It should be appreciated that the labels, instructions, and navigation scheme described above are provided by example, and that the invention may be practiced by other labels, instructions, and navigation schemes.

To operate an infrared receptive device, such as the television, toggle right on a control function. For example, toggle right on the power control function to turn the television "on" and toggle right on the power control function again to turn the television "off." Toggle right on the Ch Up control function to view the next television channel and toggle right on the Ch Dn control function to view the previous television channel. Toggle right on the Vol Up control function to turn the television volume up and toggle right on the Vol Dn control function to turn the television volume down. It should be appreciated that the labels described above are provided by example, and that the invention may be practiced by other labels.

An infrared receptive device, such as the television, may alternatively be controlled using a TV mode menu instead of the TV list menu described above by simply toggling right on the TV mode menu selection item in the IR AV control menu shown in Fig. 13E. The mode menu or feature preferably provides the most common control functions for operating the infrared receptive device. In Fig. 14, only four control functions or commands are displayed, in the example of the television, these are Ch Up, Ch Dn, Vol Up and Vol Dn. These commands are provided in the form of screen arrows, though the commands may appear in other forms. Toggle in the direction of the screen arrows to activate any of the displayed commands. Press the mode button 26a to exit the mode menu when finished. Although only four command choices are shown, it should be understood that fewer or more choices may be displayed. For example, the choices shown are displayed along the top, bottom, left and right of the menu screen, Additional choices can be displayed in the corners of the menu screen, resulting in the total of eight command choices. It should be appreciated that the labels and instructions described above are provided by example, and that other labels and instructions may be provided. It should also be appreciated that the navigation scheme described above is provided by example, and that the invention may be practiced by other navigation schemes.

An exemplary method for allowing a user to set up a control display so that the codes transmitted for a given infrared receptive device match those of the control display may be summarized, for example, as follows. For a multi digit code to be entered, the user may enter a digit of the multi digit code by incrementing or decrementing the value for each digit using, for example, forward and reverse commands on the control display. The next digit may be selected using, for example, right and left commands on the control display. Once all the digits have been entered or set, an escape command using, for example, the mode button may exit the screen. Alternatively, the screen may be exited, for example, if the wheelchair is provided with a timed function, after a period of time has lapsed, or after some other condition has been met.

An exemplary manner in which a user may more easily interact with an infrared receptive device may be summarized, by example, as follows. To make it easier to interact with the infrared receptive device, the control display may present a modal screen with a plurality of features or commands, such as buttons in the form of arrows pointing in a plurality of directions, such as in the four directions shown in Fig, 14. In Fig. 14, four arrows are shown pointing up, down, left, and right. The four arrows correspond to four directional inputs of a user input device, although other features or commands may be used. Each of the arrows may be tagged or otherwise associated with a common command associated with the infrared receptive device. When one of the directional inputs is selected, an infrared code associated with the tag is transmitted, For example, in TV mode, the display screen may present a screen as shown in Fig. 14, Selecting a forward (i.e., up arrow) command will cause a channel up infrared code to be transmitted to the television. In this way, the user need not break concentration from the infrared receptive device to scroll down a list of commands, as shown in Fig. 13F, from which to select. This modal menu will remain on the screen until an escape command is issued using, for example, the mode button, or if the wheelchair is provided with a timed function and a period of time has lapsed, or some other condition is met. For less frequently used commands, the user can select from a list, as shown in Fig. 13F, the infrared code to be sent.

The transceiver, command signal, receptive devices, functions and receiver mentioned above, are infrared by example. It should be appreciated that the invention may be practiced with other transceiver, command signal, receptive devices (i.e., environmental devices), functions and receiver.

The principle and mode of operation of this invention have been explained and illustrated in its preferred embodiment.

### REFERENCE SIGN LIST

- 10: power wheelchair
- 12: frame
- 14: driven wheel
- 16: non-driven caster wheel
- 18: drive motor
- 20: seat assembly
- 22: armrest
- 24: leg rest
- 26: user input device
- 26a: mode button
- 26b: indicator
- 26c: joystick
- 28: control display
- 28a: screen
- 30: power switch
- 32: transceiver
- 33: remote control

## Claims

1. Wheelchair (10) that controls environmental devices, the wheelchair comprising:
a chassis (12);
one or more wheels (14,16) supporting the chassis (12) for movement in relation to a supporting surface, the one or more wheels including one or more driven wheels (14), the one or more driven wheels (14) being driven by one or more drive motors (18);
a user input device (26) supported in relation to the chassis (12) wherein the user input device (26) is a hand control; and
a control display (28) supported in relation to the chassis (12) and comprising a modal screen (28a) with one or more control commands corresponding to control functions of at least one environmental device, the one or more control commands being selected by the user input device (26) to operate the environmental device, and said control display further comprising a drive display for appearing on the control display (28), the drive display comprising a ready-to-drive screen, wherein, when the ready-to-drive screen is displayed, movement of the hand control causes the wheelchair to move, **characterised in that** the one or more wheels include one or more casters (16), and **in that** a mode button is provided for exiting the modal screen and for switching between a driver menu comprising a list of shortcuts to most commonly used non-driving related menu items and the drive display.

2. Wheelchair of claim 1, wherein the user input device (26) is a hand control comprising a joystick (26c) and/or a plurality of buttons (26a).

3. Wheelchair of claim 1 or 2, wherein each of the one or more screen commands is tagged with a command associated with the environmental device so that the selection of one or more screen commands causes a transmission to the environmental device of a signal associated with the tagged command.

4. Method for setting up a wheelchair control comprising a control display (28) and a user input device (26) in the form of a hand control, in particular a wheelchair control of a wheelchair of one of the claims 2 to 4, the method comprising the steps of:
setting up the control display from a driver menu comprising a list of shortcuts to most commonly used non-driving related menu items by pressing a mode button (26a);
entering into the wheelchair control a code that matches a control code of a given environmental device; and
pressing the mode button (26a) to return to a drive display appearing on the control display (28), the drive display comprising a ready-to-drive screen, and the wheelchair being configured such that, when the ready-to-drive screen is displayed, movement of the hand control causes the wheelchair to move.

5. Method for setting up a wheelchair control of claim 4, comprising the steps of:
a) entering on a control display a code comprised of one or more digits and/or characters by searching through a string or array of digits and/or characters using at least a first control command; and
b) selecting a digit or character by using at least a second control command.

## Patentansprüche

1. Rollstuhl (10), der Umgebungsvorrichtungen steuert, wobei der Rollstuhl aufweist:
ein Chassis (12);
ein oder mehrere Räder (14,16), die das Chassis (12) zur Bewegung relativ zu einer Stützfläche stützen, wobei das eine oder die mehreren Räder ein oder mehrere angetriebene Räder (14) umfassen, wobei das eine oder die mehreren angetriebenen Räder (14) durch einen oder mehrere Antriebsmotoren (18) angetrieben werden;
eine Benutzereingabevorrichtung (26), die in Bezug auf das Chassis (12) gehalten wird, wobei die Benutzereingabevorrichtung (26) eine Handsteuerung ist; und
eine Steueranzeige (28), die in Bezug auf das Chassis (12) gehalten wird und einen Modalbildschirm (28a) mit einem oder mehreren Steuerbefehlen aufweist, die Steuerfunktionen mindestens einer Umgebungsvorrichtung entsprechen, wobei der eine oder die mehreren Steuerbefehle durch die Benutzereingabevorrichtung (26) ausgewählt werden, um die Umgebungsvorrichtung zu bedienen, und die Steueranzeige ferner eine Fahranzeige zum Erscheinen auf der Steueranzeige (28) aufweist, wobei die Fahranzeige einen Fahrbereitschaftsbildschirm aufweist, wobei, wenn der Fahrbereitschaftsbildschirm angezeigt wird, eine Bewegung der Handsteuerung bewirkt, dass sich der Rollstuhl bewegt, **dadurch gekennzeichnet, dass** das eine oder die mehreren Räder ein oder mehrere Schwenkrollen (16) aufweisen, und dass ein Betriebsartknopf zum Verlassen des Modalbildschirms und zum Umschalten zwischen einem Fahrermenü, das eine Liste von Abkürzungen für die am häufigsten verwendeten, nicht das Fahren betreffenden Menüpunkte aufweist, und der Fahranzeige vorgesehen ist.

2. Rollstuhl nach Anspruch 1, wobei die Benutzereingabevorrichtung (26) eine Handsteuerung ist, die einen Joystick (26c) und/oder mehrere Knöpfe (26a) aufweist.

3. Rollstuhl nach Anspruch 1 oder 2, wobei an jeden des einen oder der mehreren Bildschirmbefehle ein Befehl angehängt ist, der mit der Umgebungsvorrichtung verknüpft ist, so dass die Auswahl eines oder mehrerer Bildschirmbefehle eine Übertragung eines Signals, das mit dem angehängten Befehl verknüpft ist, an die Umgebungsvorrichtung bewirkt.

4. Verfahren zum Einstellen einer Rollstuhlsteuerung, die eine Steueranzeige (28) und eine Benutzereingabevorrichtung (26) in der Form einer Handsteuerung aufweist, insbesondere eine Rollstuhlsteuerung eines Rollstuhls nach einem der Ansprüche 2 bis 4, wobei das Verfahren die Schritte aufweist:
Einstellen der Steueranzeige von einem Fahrermenü, das eine Liste von Abkürzungen der am häufigsten verwendeten, nicht das Fahren betreffenden Menüpunkte aufweist, durch Drücken eines Betriebsartknopfes (26a);
Eingeben eines Codes in die Rollstuhlsteuerung, der mit einem Steuercode einer gegebenen Umgebungsvorrichtung übereinstimmt; und
Drücken des Betriebsartknopfes (26a), um zu einer Fahranzeige zurückzukehren, die auf der Steueranzeige (28) erscheint, wobei die Fahranzeige einen Fahrbereitschaftsbildschirm aufweist, und der Rollstuhl so konfiguriert ist, dass wenn der Fahrbereitschaftsbildschirm angezeigt wird, eine Bewegung der Handsteuerung bewirkt, dass sich der Rollstuhl bewegt.

5. Verfahren zum Einstellen einer Rollstuhlsteuerung nach Anspruch 4, das die Schritte aufweist:
a) Eingeben eines Codes auf einer Steueranzeige, der eine oder mehrere Ziffern und/oder Buchstaben umfasst, durch Durchsuchen einer Kette oder einer Anordnung von Ziffern und/oder Buchstaben mittels mindestens eines ersten Steuerbefehls; und
b) Auswählen einer Ziffer oder eines Buchstabens mittels mindestens eines zweiten Steuerbefehls.

## Revendications

1. Fauteuil roulant (10) commandant des dispositifs environnementaux, le fauteuil roulant comprenant :
un châssis (12) ;
une ou plusieurs roues (14, 16) supportant le châssis (12) en vue d'un déplacement par rapport à une surface porteuse, les une ou plusieurs roues comportant une ou plusieurs roues motrices (14), lesdites une ou plusieurs roues motrices (14) étant entraînées par un ou plusieurs moteurs d'entraînement (18) ;
un dispositif d'entrée utilisateur (26) supporté par rapport au châssis (12), le dispositif d'entrée utilisateur (26) étant une commande manuelle ; et
un affichage de commande (28) supporté par rapport au châssis (12) et comprenant un écran modal (28a) disposant de une ou plusieurs commandes de pilotage correspondant à des fonctions de commande d'au moins un dispositif environnemental, les une ou plusieurs commandes de pilotage étant sélectionnées par le dispositif d'entrée utilisateur (26) afin d'actionner le dispositif environnemental, et ledit affichage de commande comprenant en outre un affichage de conduite pouvant apparaître sur l'affichage de commande (28), l'affichage de conduite comprenant un écran prêt à conduire, sur lequel, lorsque l'écran prêt à conduire s'affiche, le déplacement de la commande manuelle occasionne le déplacement du fauteuil roulant, **caractérisé en ce que** les une ou plusieurs roues comprennent une ou plusieurs roulettes (16), et **en ce qu'**un bouton mode est fourni pour quitter l'écran modal et pour commuter entre un menu conduite comprenant une liste de raccourcis vers les éléments de menu les plus couramment utilisés et ne se rapportant pas à la conduite, et l'affichage de conduite.

2. Fauteuil roulant selon la revendication 1, dans lequel le dispositif d'entrée utilisateur (26) est une commande manuelle comprenant un joystick (26c) et/ou une pluralité de boutons (26a).

3. Fauteuil roulant selon la revendication 1 ou 2, dans lequel chacune de la ou des commandes d'écran est marquée avec une commande associée au dispositif environnemental, de sorte que le choix d'une ou plusieurs commandes d'écran entraîne une transmission au dispositif environnemental d'un signal associé à la commande marquée.

4. Procédé de paramétrage d'une commande de chaise roulante comprenant un affichage de commande (28) et un dispositif d'entrée utilisateur (26) sous la forme d'une commande manuelle, en particulier une commande de chaise roulante d'une chaise roulante selon l'une des revendications 2 à 4, le procédé comportant les étapes consistant à :
paramétrer l'affichage de commande à partir d'un menu conducteur comprenant une liste de raccourcis vers les éléments de menu les plus communément utilisés et ne se rapportant pas à la conduite par appui sur un bouton mode (26a) ;
saisir dans la commande de chaise roulante un code correspondant à un code de commande d'un dispositif environnemental donné ;
appuyer sur le bouton mode (26a) pour revenir à un affichage de conduite apparaissant sur l'affichage de commande (28), l'affichage de conduite comportant un écran prêt à conduire, et la chaise roulante étant configurée de telle manière que, lorsque l'écran prêt à conduire s'affiche, un déplacement de la commande manuelle entraîne le déplacement du fauteuil roulant.

5. Procédé de paramétrage d'une commande de chaise roulante selon la revendication 4, comprenant les étapes consistant à :
a) saisir sur un affichage de commande un code comportant un ou plusieurs chiffres et/ou caractères par recherche dans une chaîne ou un ensemble de chiffres et/ou de caractères en utilisant au moins une première commande de pilotage ; et
b) sélectionner un chiffre ou caractère en utilisant au moins une seconde commande de pilotage.
